# EUROPÄISCHE PATENTSCHRIFT

(11) **EP 3 276 390 B1**
(45) Veröffentlichungstag und Bekanntmachung des Hinweises auf die Patenterteilung: **03.07.2024**
(21) Anmeldenummer: 17180669.8
(22) Anmeldetag: 11.07.2017
(51) Int. Cl.: G02B 5/04, A61B 1/00, G02B 23/24

(54) **OPTISCHES SYSTEM UND CHIRURGISCHES INSTRUMENT MIT EINEM SOLCHEN OPTISCHEN SYSTEM**
OPTICAL SYSTEM AND SURGICAL INSTRUMENT WITH SUCH AN OPTICAL SYSTEM
SYSTÈME OPTIQUE ET INSTRUMENT CHIRURGICAL COMPRENANT UN TEL SYSTÈME OPTIQUE

(30) Priorität: 29.07.2016 DE 102016214025
(43) Veröffentlichungstag der Anmeldung: 31.01.2018
(73) Patentinhaber: OLYMPUS Winter & Ibe GmbH, 22045 Hamburg (DE)
(72) Erfinder: ZHAO, Jianxin, 22399 Hamburg (DE); BOCK, Mario, 22765 Hamburg (DE)
(74) Vertreter: Seemann & Partner Patentanwälte mbB

(56) Entgegenhaltungen:
- DE-A1-102010 022 430
- DE-A1-102014 211 367
- JP-A- 2000 098 263

## Beschreibung

Die Erfindung betrifft ein optisches System, umfassend eine distale optische Baugruppe, eine proximale optische Baugruppe und einen Bildsensor, wobei die distale und die proximale optische Baugruppe einen Strahlengang festlegen und die distale optische Baugruppe aus einem im Objektraum gelegenen Sichtfeld einfallende Lichtbündel in die proximale optische Baugruppe einkoppelt und die proximale optische Baugruppe diese Lichtbündel auf eine lichtempfindliche Fläche des Bildsensors lenkt. Ferner betrifft die Erfindung ein chirurgisches Instrument mit einem solchen optischen System.

Die optischen Elemente eines optischen Systems, in der Regel eine oder mehrere Linsen, bilden aus einem Sichtfeld einfallende Lichtbündel auf eine lichtempfindliche Fläche eines Bildsensors ab. Diese Lichtbündel verlaufen innerhalb eines Strahlengangs, der durch die optischen Baugruppen des optischen Systems, genauer durch deren optische Elemente, festgelegt ist.

Das Sichtfeld des optischen Systems bezeichnet einen Bereich oder einen Intervall von Bildwinkeln, innerhalb dessen Ereignisse oder Veränderungen im Objektraum mit dem optischen System wahrgenommen werden können. Aus dem Sichtfeld einfallende Lichtbündel werden auf die lichtempfindliche Fläche des Bildsensors abgebildet. Bei einem rechteckigen Bildsensor ist das Sichtfeld durch einen horizontalen Bildwinkel und durch einen vertikalen Bildwinkel definiert. Der horizontale und vertikale Bildwinkel sind durch die Ränder des Aufnahmeformats begrenzt, welches wiederum von der Größe und Form des Bildsensors abhängt. Bei einem rechteckigen Bildsensor ist der vertikale Bildwinkel typischerweise kleiner als der horizontale Bildwinkel (Querformat). Der horizontale Bildwinkel und der vertikale Bildwinkel sind also die maximal möglichen Einfallswinkel, unter denen Lichtstrahlen in das optische System einfallen können und gerade noch auf der lichtempfindlichen Fläche des Bildsensors abgebildet werden.

Fallen Lichtbündel unter größeren Winkeln in das optische System ein, kommt es zu Reflektionen an den optischen Elementen. Ebenso verursachen diese Lichtbündel diffuse Streuung oder Reflexion an einem Tubus oder Linsenrohr, in dem die optischen Elemente des optischen Systems aufgenommen sind. Diese Reflektionen, die vielfach auch als "Flare" oder "Lens Flare" bezeichnet werden, beeinflussen die Bildqualität des optischen Systems nachteilig.

Traditionell wird der Einfall solcher Lichtbündel durch mechanische Masken oder Blenden innerhalb des optischen Systems verringert. Masken oder Blenden führen jedoch vielfach zu einer starken Vignettierung, also einer Abschattung zum Bildrand hin. Ferner setzen solche Masken sehr enge Fertigungstoleranzen für das optische System voraus.

DE 10 2014 211 367 A1 offenbart ein Endoskop und eine Prismenvorrichtung für ein Endoskop. Ein Prisma der Prismenvorrichtung umfasst zumindest eine Reflexionsfläche, die eine Grenzfläche zu einem gasförmigen optisch dünneren Medium darstellt. Eine Abdeckung an der Außenseite schließt ein Volumen des gasförmigen optisch dünneren Mediums ein.

In DE 10 2010 022 430 A1 ist eine Sichtfeldvorrichtung für eine Bildübertragungsvorrichtung offenbart, die einen Kanal umfasst, der das proximale Ende und das distale Ende der Sichtfeldvorrichtung verbindet. Eine optische Einrichtung ist am distalen Ende des Kanals ausgebildet, um das Sichtfeld der Bildübertragungsvorrichtung zu beeinflussen.

JP 2000 098263 A1 zeigt ein Schrägsichtendoskop mit einem Prisma, das einfallendes Licht in eine Linsengruppe einkoppelt.

Es ist eine Aufgabe der Erfindung, ein verbessertes optisches System sowie ein verbessertes chirurgisches Instrument mit einem solchen optischen System bereitzustellen, wobei das optisches System insbesondere unempfindlicher gegenüber von außerhalb eines Bildfeldes einfallenden Lichtbündeln sein soll.

Die Aufgabe wird gelöst durch ein optisches System, umfassend eine distale optische Baugruppe, eine proximale optische Baugruppe und einen Bildsensor, wobei die distale und die proximale optische Baugruppe einen Strahlengang festlegen und die distale optische Baugruppe aus einem im Objektraum gelegenen Sichtfeld einfallende Lichtbündel in die proximale optische Baugruppe einkoppelt und die proximale optische Baugruppe diese Lichtbündel auf eine lichtempfindliche Fläche des Bildsensors lenkt, wobei das optische System fortgebildet ist durch eine im Strahlengang angeordnete Prismengruppe mit zumindest einem Prisma, wobei die Prismengruppe das Sichtfeld des optischen Systems an zumindest einer Seite begrenzt, wobei das zumindest eine Prisma eine Grenzfläche umfasst, an der von außerhalb des Sichtfeldes einfallende Lichtstrahlen unter Totalreflexion aus dem Strahlengang herausreflektiert werden, wobei die Prismengruppe zumindest ein erstes und ein zweites Prisma umfasst, wobei das erste Prisma die Grenzfläche, an der Totalreflexion stattfindet, umfasst und das zweite Prisma angrenzend an diese Grenzfläche angeordnet ist, wobei zwischen dem ersten Prisma und dem zweiten Prisma ein erster Luftspalt vorhanden ist.

Vorteilhaft stellt die Prismengruppe einen winkelabhängigen optischen Filter bereit, mit dem von außerhalb des Sichtfeldes in das optische System einfallende Lichtstrahlen aus dem Strahlengang herausreflektiert werden. Es tritt keine Vignettierung auf und es sind auch keine besonders hohen Anforderungen an die Zentrierung oder Justage der Prismengruppe im optischen System zu stellen. Eine Prismengruppe ist außerdem preisgünstig herzustellen und kann ohne großen konstruktiven Aufwand in das optische System integriert werden.

Es ist insbesondere vorgesehen, dass die Prismengruppe im optischen System drehbar aufgenommen ist. Beispielsweise ist die Prismengruppe innerhalb des optischen Systems um eine Achse drehbar, welche zumindest näherungsweise der optischen Achse des optischen Systems, insbesondere einer optischen Achse der distalen optischen Baugruppe des optischen Systems, entspricht. Durch eine Drehung der Prismengruppe ist es vorteilhaft möglich, das Sichtfeld variabel an verschiedenen Seiten mithilfe der Prismengruppe zu begrenzen.

Das zumindest eine Prisma umfasst eine Grenzfläche, an der von außerhalb des Sichtfeldes einfallende Lichtstrahlen unter Totalreflexion aus dem Strahlengang herausreflektiert werden. Bei dieser Grenzfläche handelt es sich um eine Grenzfläche von einem optisch dichten Medium zu einem optisch dünneren Medium. Beispielsweise handelt es sich um eine Grenzfläche Glas-Luft.

Dabei ist insbesondere vorgesehen, dass die herausreflektierten Lichtstrahlen an einer Seite des Sichtfeldes in die distale optische Baugruppe einfallen, an der die Prismengruppe das Sichtfeld begrenzt.

Die Prismengruppe umfasst zumindest ein erstes und ein zweites Prisma, wobei das erste Prisma die Grenzfläche, an der Totalreflexion stattfindet, umfasst, und das zweite Prisma angrenzend an diese Grenzfläche angeordnet ist, wobei zwischen dem ersten Prisma und dem zweiten Prisma ein erster Luftspalt vorhanden ist.

Mit anderen Worten sind also das erste Prisma und das zweite Prisma unmittelbar benachbart zueinander angeordnet. Nicht im beanspruchten Schutzumfang ist alternativ, zu einem Luftspalt zwischen dem ersten Prisma und dem zweiten Prisma ist eine Verbindung der beiden Prismen an der Grenzfläche, an der Totalreflexion stattfindet, vorgesehen, wobei zur Verbindung der beiden Prismen ein Material verwendet wird, welches optisch eine geringere Dichte als das Material des ersten Prismas aufweist.

Totalreflexion findet beim Auftreffen von Licht an einer Grenzfläche statt, welche zwischen einem optisch dichteren und einem optisch dünneren Medium liegt. Der totalreflektierte Lichtstrahl trifft dabei unter einem Winkel auf die Grenzfläche auf, der größer als der für die gegebene Materialpaarung kritische Winkel ist. Die Winkel werden in Bezug auf ein senkrecht auf der Grenzfläche stehendes Lot betrachtet. Der totalreflektierte Strahl breitet sich stets im optisch dichteren Medium aus. Eine Grenzfläche Glas-Luft stellt eine sehr einfache Möglichkeit dar, um diese Situation, in der ein Lichtstrahl totalreflektiert wird, bereitzustellen.

Gemäß einer weiteren Ausführungsform ist das optische System dadurch fortgebildet, dass die Prismengruppe zusätzlich ein drittes Prisma umfasst, wobei das erste bis dritte Prisma spiegelsymmetrisch zu einer senkrecht zu einer optischen Achse der Prismengruppe gelegenen Mittenebene aufgebaut ist.

Ein vorteilhaftes spiegelsymmetrisches System umfasst eine Prismengruppe, die in Lichteinfallsrichtung hintereinander ein erstes rechtwinkliges Prisma als erstes Prisma, ein gleichschenkliges Prisma als zweites Prisma und ein zweites rechtwinkliges Prisma als drittes Prisma umfasst. Das zweite Prisma umfasst eine zweite Grenzfläche, an der Totalreflexion stattfindet. Das dritte Prisma ist angrenzend an diese zweite Grenzfläche angeordnet, wobei zwischen dem zweiten Prisma und dem dritten Prisma ein zweiter Luftspalt vorhanden ist.

Auch anstatt des zweiten Luftspalts kann ein Material vorgesehen werden, welches eine optisch geringere Dichte als das Material des zweiten Prismas aufweist.

Durch Reflexion an der ersten und an der zweiten Grenzfläche wird vorteilhaft das Sichtfeld an zwei Seiten begrenzt. Gemäß weiteren Ausführungsbeispielen ist es durch Hinzufügen weiterer Prismen möglich, das Sichtfeld auch an weiteren Seiten zu begrenzen.

Es ist insbesondere vorgesehen, dass die erste und die zweite Grenzfläche in unterschiedliche Richtungen geneigt sind, so dass aus dem Strahlengang herausreflektierte Strahlenbündel den Strahlengang in auch unterschiedliche Richtungen verlassen.

Dabei sind die erste und die zweite Grenzfläche insbesondere um eine erste und eine zweite Achse geneigt. Die erste und zweite Achse liegen zumindest näherungsweise parallel zueinander oder weisen in senkrecht aufeinander stehende Richtungen. Die Grenzflächen, an denen Totalreflexion stattfindet, sind beispielsweise parallel zu den Seiten des gleichschenkligen Prismas, welches als zweites Prisma wirkt, ausgerichtet. Dabei wird die erste Grenzfläche von einer Oberfläche des ersten Prismas bereitgestellt, während die zweite Grenzfläche von einer Oberfläche des zweiten Prismas bereitgestellt wird.

Das optische System ist gemäß einer weiteren Ausführungsform dadurch fortgebildet, dass das zumindest eine Prisma der Prismengruppe ein gerades Prisma ist. Das zumindest eine Prisma umfasst zwei optisch wirksame Prismenflächen, die einen spitzen Winkel einschließen, und eine dem spitzen Winkel gegenüberliegende, optisch nicht wirksame Fläche. Eine erste Ebene, in der eine erste optisch wirksame Prismenfläche liegt, eine zweite Ebene, in der eine zweite optisch wirksame Prismenfläche liegt, und eine dritte Ebene, in der die Fläche liegt, schließen gemeinsam ein Dreieck ein, welches zumindest bereichsweise eine Grundfläche des Prismas ist.

Mit anderen Worten schließen also die erste bis dritte Ebene das Dreieck ein. Unter einer optisch wirksamen Grenzfläche wird eine solche Grenzfläche verstanden, die im Strahlengang liegt. Die Grundfläche des Prismas ist nicht notwendigerweise dreieckig. Es handelt sich beispielsweise um einen Keil oder um ein Dreieck, dessen Spitze gekappt ist.

Gemäß einer weiteren vorteilhaften Ausführungsform ist vorgesehen, dass die Prismengruppe zumindest ein erstes Prisma und ein zweites Prisma umfasst, wobei das erste und das zweite Prisma so angeordnet sind, dass ein erster spitzer Winkel des ersten Prismas und ein zweiter spitzer Winkel des zweiten Prismas auf einander gegenüberliegenden Seiten der Prismengruppe liegen.

Der erste spitze Winkel und der zweite spitze Winkel sind also mit anderen Worten in einander entgegengesetzter Richtung ungeöffnet.

Dabei ist insbesondere vorgesehen, dass das erste und das zweite Prisma in Lichteinfallsrichtung hintereinander angeordnet sind, wobei das erste Prisma ein rechtwinkliges Prisma ist. Diese Ausführungsform der Prismengruppe ist besonders einfach zu realisieren und gleichzeitig optisch sehr effizient.

Gemäß einer weiteren vorteilhaften Ausführungsform ist das optische System dadurch fortgebildet, dass die Prismengruppe ein Teil der distalen optischen Baugruppe ist, wobei insbesondere die distale optische Baugruppe eine Eintrittslinse umfasst und die Prismengruppe in Lichteinfallsrichtung unmittelbar auf die Eintrittslinse folgend angeordnet ist.

Diese Anordnung der Prismengruppe ist besonders vorteilhaft, da von außerhalb des Sichtfeldes einfallende Lichtstrahlen, welche innerhalb des optischen Systems Streulichteffekte hervorrufen, bereits am Anfang des optischen Systems aus dem Strahlengang entfernt werden. Dies trägt erheblich zur Verbesserung der Bildqualität des optischen Systems bei.

Außerdem ist ein solcher Aufbau vorteilhaft, da die proximale optische Baugruppe von den getroffenen Maßnahmen zur Verbesserung der Bildqualität komplett unbeeinflusst ist. Das optische System ist also im Hinblick auf das Design der proximalen optischen Baugruppe sehr flexibel.

Das optische System umfasst insbesondere zumindest einen Bildsensor. Es handelt sich bei dem optischen System ferner insbesondere um ein optisches System zur Erfassung stereoskopischer Bilddaten.

So ist gemäß einem weiteren Ausführungsbeispiel vorgesehen, dass die proximale optische Baugruppe einen linken Linsensystemkanal mit einer linken optischen Achse und einen rechten Linsensystemkanal mit einer rechten optischen Achse umfasst. Der linke und der rechte Linsensystemkanal sind gleichartig aufgebaut und die linke und die rechte optische Achse sind parallel zueinander ausgerichtet. Die distale optische Baugruppe ist dazu eingerichtet, das aus dem Objektraum einfallende Licht sowohl in dem linken Linsensystemkanal als auch in den rechten Linsensystemkanal der proximalen optischen Baugruppe einzukoppeln.

Ein solches optisches System ist insbesondere zur Verwendung in einem Stereo-Videoendoskop geeignet.

Die Aufgabe wird ferner gelöst durch ein chirurgisches Instrument, insbesondere ein Endoskop, ferner insbesondere ein Stereo-Videoendoskop, mit einem optischen System nach einem oder mehreren der zuvor genannten Ausführungsformen.

Das optische System ist insbesondere das optische System eines chirurgischen Instruments, ferner insbesondere das optische System eines Stereo-Videoendoskops.

Auf das chirurgische Instrument, insbesondere auf das Endoskop, treffen gleiche oder ähnliche Vorteile zu, wie sie bereits im Hinblick auf das optische System selbst erwähnt wurden. Besonders vorteilhaft kann ein optisches System mit hoher Streulichtunempfindlichkeit angegeben werden, welches außerdem effizient und einfach herstellbar ist.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: ein optisches System in einem schematisch vereinfachten Längsschnitt, gemäß dem Stand der Technik,
- Fig. 2: ein optisches System gemäß einem Ausführungsbeispiel, in einem schematisch vereinfachten Längsschnitt,
- Fig. 3: eine Prismengruppe des optischen Systems aus Fig. 2 in einem schematisch vereinfachten Längsschnitt,
- Fig. 4: eine weitere Darstellung der Prismengruppe aus Fig. 3 in einem schematisch vereinfachten Längsschnitt,
- Fig. 5: ein weiteres optisches System gemäß dem Stand der Technik, wobei von dessen proximaler optischer Baugruppe lediglich ein linker Linsensystemkanal dargestellt ist, in einem schematisch vereinfachten Längsschnitt, und
- Fig. 6: ein optisches System eines Stereo-Videoendoskops gemäß einem Ausführungsbeispiel, umfassend eine Prismengruppe, wobei von der proximalen optischen Baugruppe lediglich der linke Linsensystemkanal dargestellt ist.

In den Zeichnungen sind jeweils gleiche oder gleichartige Elemente und/oder Teile mit denselben Bezugsziffern versehen, so dass von einer erneuten Vorstellung jeweils abgesehen wird.

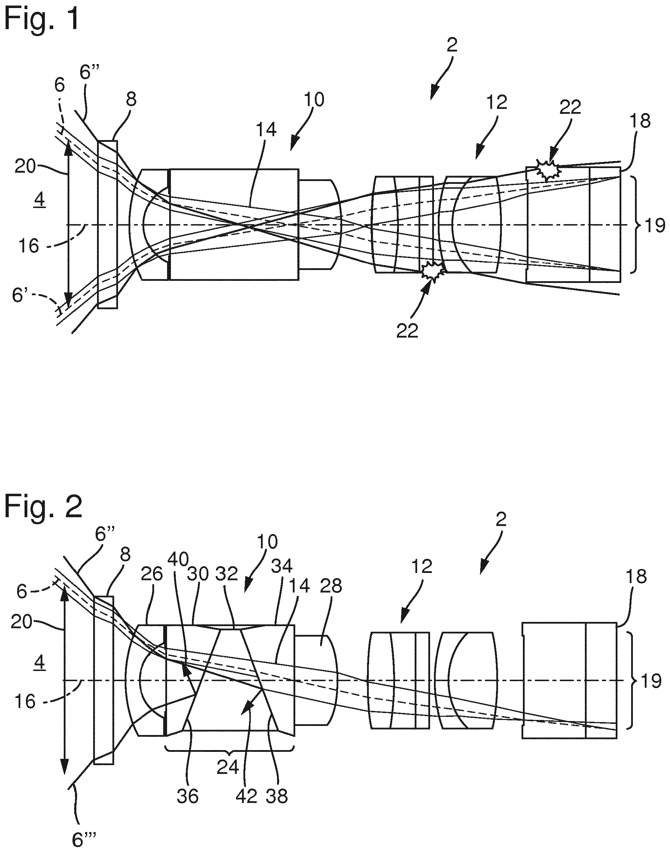

Fig. 1 zeigt ein optisches System 2 gemäß dem Stand der Technik in einer schematisch vereinfachten Längsschnittdarstellung. Aus einem Objektraum 4 einfallende Lichtbündel 6 (von denen aus Gründen der Übersichtlichkeit lediglich eines mit Bezugszeichen versehen ist) treffen zunächst auf ein Eintrittsfenster 8. Das optische System 2 ist beispielsweise ein Bauteil eines chirurgischen Instruments, ferner beispielsweise das optische System 2 eines Endoskops. Bei einem Endoskop schließt das Eintrittsfenster 8 den Innenraum eines Endoskopschafts an seinem distalen Ende gegenüber einem Außenraum bzw. Objektraum 4 hermetisch ab. Haben die Lichtbündel 6 das Eintrittsfenster 8 durchquert, treffen sie auf eine distale optische Baugruppe 10 und gelangen anschließend in eine proximale optische Baugruppe 12. Die distale und die proximale optische Baugruppe 10, 12 definieren einen Strahlengang 14 innerhalb des optischen Systems 2.

Im Objektraum 4 ist ein Sichtfeld 20 gelegen, welches durch einen horizontalen und einen vertikalen Bildwinkel definiert ist. Der in Fig. 1 gezeigte Längsschnitt zeigt beispielhaft einen Schnitt entlang einer vertikalen Ebene. Folglich ist der vertikale Bildwinkel zu sehen. Es handelt sich um den Winkel zwischen einer optischen Achse 16 des optischen Systems 2 und dem Lichtbündel 6, welches gerade noch auf eine lichtempfindliche Fläche 19 eines Bildsensors 18 trifft. Das Sichtfeld 20 ist in Fig. 1 schematisch mit einem Pfeil angedeutet. Die distale optische Baugruppe 10 und die proximale optische Baugruppe 12 bilden aus dem Sichtfeld 20 einfallende Lichtbündel 6, 6' auf die lichtempfindliche Fläche 19 des Bildsensors 18 ab.

Fallen jedoch Lichtbündel 6" von außerhalb des Sichtfeldes 20 in das optische System 2 ein, so verursachen diese diffuse Streuung und Reflexionen innerhalb des optischen Systems 2. Beispielsweise kommt es zu diffuser Streuung an einer Innenwand eines Tubus oder eines Linsenrohrs des optischen Systems 2. Dies ist in Fig. 1 mit sternförmigen Markierungen angedeutet, welche Streuzentren 22 andeuten sollen. Diese Reflexionen oder Streuungen verursachen "Flare" oder "Lense Flare", ein Phänomen, welches die Bildqualität des optischen Systems 2 nachteilig beeinflusst.

Fig. 2 zeigt ein optisches System 2 gemäß einem Ausführungsbeispiel, ebenfalls in einer vereinfachten und schematischen Längsschnittansicht entlang einer vertikalen Schnittebene. Das optische System 2 umfasst eine distale optische Baugruppe 10 sowie eine proximale optische Baugruppe 12. Die distale optische Baugruppe 10 und die proximale optische Baugruppe 12 definieren innerhalb des optischen Systems 2 einen Strahlengang 14. Aus dem Objektraum 4 von innerhalb des Sichtfeldes 20 in das optische System 2 einfallende Lichtbündel 6 (von denen beispielhaft lediglich eines dargestellt ist) werden auf eine lichtempfindliche Fläche 19 des Bildsensors 18 abgebildet.

Das optische System 2 gemäß dem dargestellten Ausführungsbeispiel umfasst eine im Strahlengang 14 angeordnete Prismengruppe 24. Die Prismengruppe 24 umfasst zumindest ein Prisma 30, 32, 34 und begrenzt das Sichtfeld 20 des optischen Systems 2 an zumindest einer Seite. Neben der Prismengruppe 24 umfasst die distale optische Baugruppe 10 noch eine Eintrittslinse 26 und eine Austrittslinse 28. Das zumindest eine Prisma 30, 32, 34 der Prismengruppe 24 umfasst eine Grenzfläche 36, 38, an der von außerhalb des Sichtfeldes 20 in das optische System 2 einfallende Lichtstrahlen 6" unter Totalreflexion aus dem Strahlengang 14 herausreflektiert werden.

Die in Fig. 2 gezeigte Prismengruppe 24 umfasst beispielhaft ein erstes Prisma 30, ein zweites Prisma 32 und ein drittes Prisma 34. Das erste Prisma 30 stellt eine erste Grenzfläche 36 bereit, an der ein erstes Lichtbündel 40 (angedeutet durch einen Pfeil) aus dem Strahlengang 14 herausreflektiert wird. Das zweite Prisma 32 stellt eine zweite Grenzfläche 36 bereit, an der ein zweites Lichtbündel 42 (ebenfalls angedeutet durch einen Pfeil) in einer anderen Richtung aus dem Strahlengang 14 herausreflektiert wird.

Die aus dem Strahlengang 14 herausreflektierten Lichtbündel treten von außerhalb des Sichtfeldes 20 als Lichtbündel 6" und 6‴ in das optische System 2 ein. Das in Fig. 2 an der Unterseite des optischen Systems 2 von außerhalb des Sichtfeldes 20 einfallende Lichtbündel 6‴ wird als erstes Lichtbündel 40 an der ersten Grenzfläche 36 totalreflektiert und so aus dem Strahlengang 14 entfernt. Das von außerhalb des Sichtfeldes 20 an der oberen Seite in das optische System 2 einfallende Lichtbündel 6" wird als zweites Lichtbündel 42 an der zweiten Grenzfläche 38 totalreflektiert und auf diese Weise aus dem Strahlengang 14 herausreflektiert.

Die Prismengruppe 24 begrenzt das Sichtfeld 20 an zwei einander gegenüberliegenden Seiten, beispielhaft an einer unteren und an einer oberen horizontalen Kante des Sichtfeldes 20. An diesen Seiten des Sichtfeldes 20 in das optische System 2 einfallende Lichtstrahlen 6", 6‴, welche von außerhalb des Sichtfeldes 20 einfallen, werden aus dem Strahlengang 14 herausreflektiert. Durch eine Drehung der Prismengruppe 24 um die optische Achse 16 kann in gleicher Weise eine Begrenzung, beispielsweise an den vertikalen Kanten des Sichtfeldes 20, erfolgen als an der linken oder der rechten Seite des Sichtfeldes 20. Hierzu müsste die Prismengruppe 24 um 90° um die optische Achse 16 gedreht werden, außerdem müsste sie auf den erforderlichen horizontalen Blickwinkel (der möglicherweise größer ist als der vertikale Blickwinkel) angepasst werden. Eine solche Anpassung erfolgt beispielsweise durch geeignete Wahl der Neigung der Grenzflächen 36, 38 gegenüber der optischen Achse 16.

Es ist ebenso möglich, eine weitere, in Fig. 2 nicht dargestellte, Prismengruppe 24 hinzuzufügen. Bei einem solchen Ausführungsbeispiel wäre eine erste Prismengruppe 24 wie die in Fig. 2 dargestellte Prismengruppe 24 angeordnet, eine zweite Prismengruppe wäre in Lichteinfallsrichtung folgend um 90° um die optische Achse 16 gedreht angeordnet. So könnte eine Begrenzung des Sichtfeldes 20 sowohl an den horizontalen als auch an den vertikalen Grenzen des Sichtfeldes 20 erreicht werden.

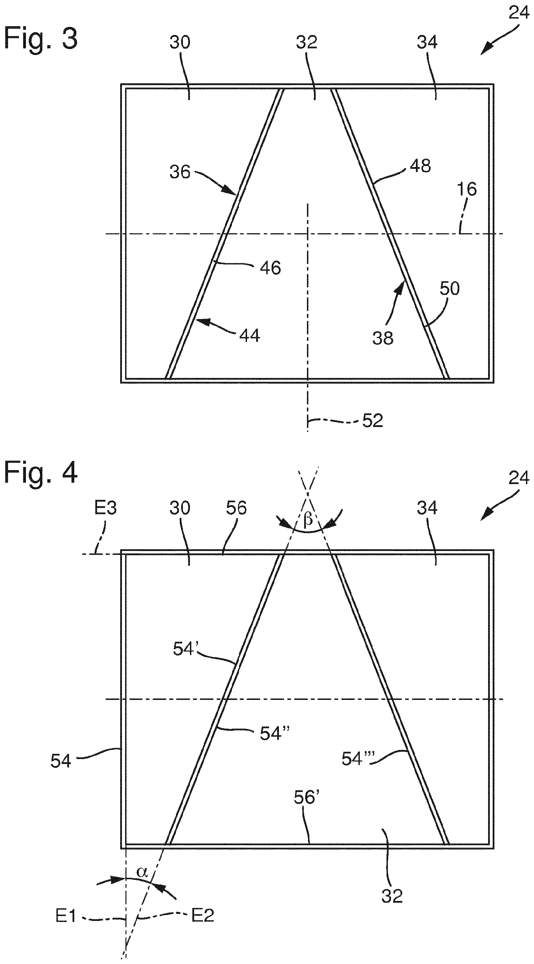

Fig. 3 zeigt die aus Fig. 2 bekannte Prismengruppe 24, ebenfalls in einem schematisch vereinfachten vertikalen Längsschnitt. Die Prismengruppe 24 ist spiegelsymmetrisch zu einer abschnittsweise angedeuteten Mittenebene 52 ausgebildet. Die Mittenebene 52 steht senkrecht auf der optischen Achse 16. Die Prismen 30, 32, 34 der Prismengruppe 24 sind bevorzugt gerade Prismen.

Das erste Prisma 30 der Prismengruppe 24 und ebenso das dritte Prisma 34 sind rechtwinklige Prismen. Das zweite Prisma 32 ist ein gleichschenkliges Prisma. Das erste Prisma 30 umfasst die erste Grenzfläche 36, an der Totalreflexion stattfindet. Zwischen dieser ersten Grenzfläche 36 und einer Eintrittsfläche 44 des zweiten Prismas 32 befindet sich ein erster Luftspalt 46. Zwischen einer zweiten Grenzfläche 38, welche eine Austrittsfläche des zweiten Prismas 32 ist, und einer weiteren Eintrittsfläche 48 des dritten Prismas 34 befindet sich ein zweiter Luftspalt 50. Sowohl an der ersten Grenzfläche 36 als auch an der zweiten Grenzfläche 38 findet jeweils ein Übergang von einem optisch dichteren Medium, nämlich dem Material des ersten Prismas 30 bzw. des zweiten Prismas 32, bei dem es sich beispielsweise um Glas handelt, zu einem optisch dünneren Medium, nämlich der Luft im jeweiligen Luftspalt 46, 50, statt. An der ersten und zweiten Grenzfläche 36, 38 werden die Lichtbündel 40, 42 (vgl. Fig. 2) totalreflektiert.

Die Prismen 30, 32, 34 sind zueinander angrenzend angeordnet. Es befinden sich also keine weiteren optischen Elemente zwischen den Prismen 30, 32, 34; die Prismengruppe 24 umfasst keine weiteren optischen Elemente. Insbesondere ist die Eintrittsfläche 44 des zweiten Prismas 32 angrenzend zu der ersten Grenzfläche 36 des ersten Prismas 30 angeordnet. Zwischen diesen beiden Grenzflächen 36, 44 befindet sich lediglich der erste Luftspalt 46. Gleiches gilt für die Anordnung des zweiten und dritten Prismas 32, 34. Auch hier ist die weitere Eintrittsfläche 48 des dritten Prismas 34 angrenzend zu der zweiten Grenzfläche 38 angeordnet. Zwischen diesen beiden Flächen 38, 48 befindet sich lediglich der zweite Luftspalt 50. Anstatt der Luftspalte 46, 50 ist gemäß weiterer Ausführungsbeispiele vorgesehen, dass die Spalte mit einem optisch dünneren Medium gefüllt werden. Entscheidend ist, dass die optische Dichte dieses Mediums geringer ist als die optische Dichte des Materials des ersten Prismas 30, im Fall des ersten Luftspaltes 46, und optisch dünner als das Material des zweiten Prismas 32, im Fall des zweiten Luftspaltes 50. Beispielsweise können die Prismen 30, 32, 34 miteinander verkittet werden.

Die erste Grenzfläche 36 und die zweite Grenzfläche 38 sind in unterschiedliche Richtungen geneigt. Dadurch werden die aus dem Strahlengang 14 herausreflektierten Strahlenbündel 40, 42 in unterschiedliche Richtungen aus diesem herausreflektiert. Aufgrund der symmetrischen Ausbildung der Prismengruppe 24 sind die Grenzflächen 36, 38 um jeweils einen gleichen Winkel geneigt. Außerdem erfolgt ihre Neigung um zueinander parallele Achsen.

Fig. 4 zeigt eine weitere schematische Darstellung der Prismengruppe 24. Das erste Prisma 30 umfasst zwei optisch wirksame Prismenflächen 54, 54', die einen spitzen Winkel α einschließen. Ferner umfasst das erste Prisma 30 eine dem spitzen Winkel α gegenüberliegende, optisch nicht wirksame Fläche 56. Die erste optisch wirksame Prismenfläche 54 liegt in einer ersten Ebene E1 (durch gestrichelte Linie angedeutet). Die zweite optisch wirksame Prismenfläche 54' liegt in einer zweiten Ebene E2. Die Fläche 56 liegt in einer dritten Ebene E3. Die erste bis dritte Ebene E1, E2, E3 schließen ein Dreieck ein, welches zumindest bereichsweise eine Grundfläche des geraden ersten Prismas 30 ist. Die tatsächliche Grundfläche des Prismas 32 ist ein Dreieck, dessen Spitze gekappt ist. Für das zweite und dritte Prisma 32, 34 können entsprechende Konstruktionen gefunden werden. Die jeweils zwei optisch wirksamen Prismenflächen schließen ebenso einen spitzen Winkel ein, der einer weiteren nicht optisch wirksamen Fläche des Prismas 32, 34 gegenüberliegt. Diese Flächen liegen in Ebenen, die ein Dreieck einschließen, welches abschnittsweise die Grundfläche der Prismen bildet. Beispielsweise umfasst die Prismengruppe 24 neben dem ersten Prisma 30 das zweite Prisma 32, welches in diesem Sinne ebenso wie das erste Prisma 30 ausgestaltet ist. Auch das zweite Prisma 32 umfasst optisch wirksame Prismenflächen 54", 54‴, welche einer Fläche 56' gegenüber liegen und einen spitzen Winkel β einschließen.

Das erste und das zweite Prisma 30, 32 sind so angeordnet, dass der erste spitze Winkel α des ersten Prismas 30 und der zweite spitze Winkel β des zweiten Prismas 32 auf einander gegenüberliegenden Seiten der Prismengruppe 24 liegen. Die spitzen Winkel α, β liegen also einander gegenüber.

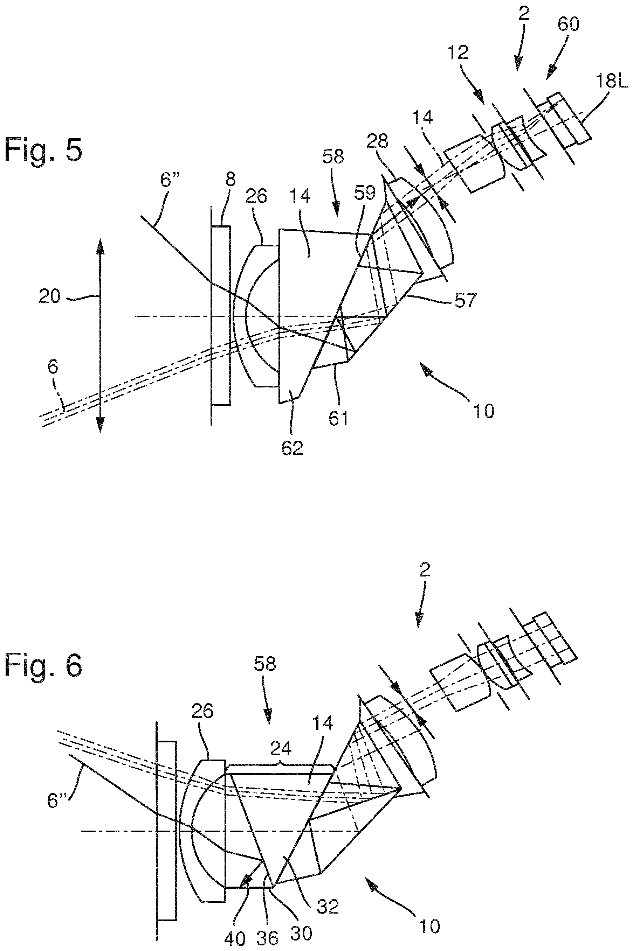

Die zuvor beschriebenen Konstruktionsprinzipien gelten ebenso für die Prismengruppe 24 des Ausführungsbeispiels in Fig. 6, auf welche weiter unten im Detail eingegangen werden soll.

Zunächst zeigt Fig. 5 ein weiteres optisches System 2 gemäß dem Stand der Technik. Das optische System 2 kommt beispielsweise in einem Stereo-Video-Endoskop mit seitlicher Blickrichtung zum Einsatz. Es befindet sich im Endoskopschaft hinter einem Eintrittsfenster 8, durch welches Lichtbündel 6 aus dem Sichtfeld 20 in das optische System 2 eintreten. Die Lichtbündel 6 gelangen zunächst in die distale optische Baugruppe 10, die eine Eintrittslinse 26, eine Umlenkprismengruppe 58 sowie eine Austrittslinse 28 umfasst. Eine proximale optische Baugruppe 12 des gezeigten optischen Systems 2 umfasst einen linken und rechten Linsensystemkanal. Beispielhaft ist in Fig. 5 lediglich der linke Linsensystemkanal 60 gezeigt. In dem linken Linsensystemkanal 60 befindet sich ein linker Bildsensor 18L. Aus dem Sichtfeld 20 in das optische System 2 einfallende Lichtbündel 6 werden von der distalen optischen Baugruppe 10 und von der proximalen optischen Baugruppe 12 innerhalb eines Strahlengangs 14 auf den linken Bildsensor 18L und einen nicht dargestellten rechten Bildsensor abgebildet. Ein von außerhalb des Sichtfeldes 20 einfallendes Lichtbündel 6" verursacht in dem optischen System 2 durch Vierfachreflexion ein Geisterbild. Das Lichtbündel wird zweimal an einer Hinterseite 57 und zweimal an einer Vorderseite 59 eines zweiten Umlenkprismas 61 der Umlenkprismengruppe 58 reflektiert.

Fig. 6 zeigt ein weiteres optisches System 2 gemäß einem Ausführungsbeispiel. Das optische System 2 ist beispielsweise das optische System 2 eines Stereo-Video-Endoskops. Das optische System 2 umfasst als Teil der Umlenkprismengruppe 58 eine Prismengruppe 24, die eine Grenzfläche 36 umfasst, an der das von außerhalb des Sichtfeldes 20 einfallende Lichtbündel 6" als erstes Lichtbündel 40 aus dem Strahlengang 14 herausreflektiert wird. Zu diesem Zweck umfasst die Prismengruppe 24 das erste Prisma 30 und das zweite Prisma 32. Zwischen der ersten Grenzfläche 36 des ersten Prismas 30 ist erneut bevorzugt ein erster Luftspalt vorgesehen, so dass Totalreflexion an der Grenzfläche 36 stattfindet. Das erste und das zweite Prisma 30, 32 sind insbesondere so ausgestaltet, dass diese das erste Umlenkprisma 62 der Umlenkprismengruppe 58 ersetzen, also eine äquivalente optische Wirkung (abgesehen von der Totalreflexion von nicht aus dem Sichtfeld 20 kommenden Lichtbündeln 6") entfalten.

Besonders vorteilhaft ist die Anordnung der Prismengruppe 24 unmittelbar anschließend an die Eintrittslinse 26. Dies gilt für das Ausführungsbeispiel in Fig. 2 und in Fig. 6. Die Prismengruppe 24, welche jeweils ein Teil der distalen optischen Baugruppe 10 ist, entfernt unerwünschtes Streulicht direkt am Anfang des optischen Systems 2. Dies steigert die Abbildungsqualität des optischen Systems 2.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezugszeichenliste

- 2: optisches System
- 4: Objektraum
- 6, 6', 6", 6‴: Lichtbündel
- 8: Eintrittsfenster
- 10: distale optische Baugruppe
- 12: proximale optische Baugruppe
- 14: Strahlengang
- 16: optische Achse
- 18: Bildsensor
- 19: lichtempfindliche Fläche
- 20: Sichtfeld
- 22: Streuzentrum
- 24: Prismengruppe
- 26: Eintrittslinse
- 28: Austrittslinse
- 30: erstes Prisma
- 32: zweites Prisma
- 34: drittes Prisma
- 36: erste Grenzfläche
- 38: zweite Grenzfläche
- 40: erstes Lichtbündel
- 42: zweites Lichtbündel
- 44: Eintrittsfläche
- 46: erster Luftspalt
- 48: weitere Eintrittsfläche
- 50: zweiter Luftspalt
- 52: Mittenebene
- 54, 54': optisch wirksame Prismenfläche
- 56: Fläche
- 57: Hinterseite
- 58: Umlenkprismengruppe
- 59: Vorderseite
- 60: linker Linsensystemkanal
- 61: zweites Umlenkprisma
- 62: erstes Umlenkprisma

- E1, E2, E3: Ebene

## Patentansprüche

1. Optisches System (2), umfassend eine distale optische Baugruppe (10), eine proximale optische Baugruppe (12) und einen Bildsensor (18), wobei die distale und die proximale optische Baugruppe (10, 12) einen Strahlengang (14) festlegen und die distale optische Baugruppe (10) aus einem im Objektraum (4) gelegenen Sichtfeld (20) einfallende Lichtbündel (6, 6') in die proximale optische Baugruppe (12) einkoppelt und die proximale optische Baugruppe (12) diese Lichtbündel (6, 6') auf eine lichtempfindliche Fläche (19) des Bildsensors (18) lenkt, **gekennzeichnet durch** eine im Strahlengang (14) angeordnete Prismengruppe (24) mit zumindest einem Prisma (30, 32, 34), wobei die Prismengruppe (24) das Sichtfeld (20) des optischen Systems (2) an zumindest einer Seite begrenzt, wobei das zumindest eine Prisma (30, 32, 34) eine Grenzfläche (36, 38) umfasst, an der von außerhalb des Sichtfeldes (20) einfallende Lichtstrahlen (6", 6‴) unter Totalreflexion aus dem Strahlengang (14) herausreflektiert werden, wobei die Prismengruppe (24) zumindest ein erstes und ein zweites Prisma (30, 32) umfasst, wobei das erste Prisma (30) die Grenzfläche (36), an der Totalreflexion stattfindet, umfasst und das zweite Prisma (32) angrenzend an diese Grenzfläche (36) angeordnet ist, wobei zwischen dem ersten Prisma (30) und dem zweiten Prisma (32) ein erster Luftspalt (46) vorhanden ist.

2. Optisches System (2) nach Anspruch 1, **dadurch gekennzeichnet, dass** die herausreflektierten Lichtstrahlen (40, 42) an einer Seite des Sichtfeldes (20) in die distale optische Baugruppe (10) einfallen, an der die Prismengruppe (24) das Sichtfeld (20) begrenzt.

3. Optisches System (2) nach Anspruch 1 oder 2, **dadurch gekennzeichnet, dass** die Prismengruppe (24) zusätzlich ein drittes Prisma (34) umfasst, wobei das erste bis dritte Prisma (30, 32, 34) spiegelsymmetrisch zu einer senkrecht zu einer optischen Achse (16) der Prismengruppe (24) gelegenen Mittenebene (52) aufgebaut ist.

4. Optisches System (2) nach Anspruch 3, **dadurch gekennzeichnet, dass** die Prismengruppe (24) in Lichteinfallsrichtung hintereinander ein erstes rechtwinkliges Prisma als erstes Prisma (30), ein gleichschenkliges Prisma als zweites Prisma (32) und ein zweites rechtwinkliges Prisma als drittes Prisma (34) umfasst, wobei das zweite Prisma (32) eine zweite Grenzfläche (38) umfasst, an der Totalreflexion stattfindet, und das dritte Prisma (34) angrenzend an diese zweite Grenzfläche (38) angeordnet ist, wobei zwischen dem zweiten Prisma (32) und dem dritten Prisma (34) ein zweiter Luftspalt (50) vorhanden ist.

5. Optisches System (2) nach Anspruch 4, **dadurch gekennzeichnet, dass** die erste und die zweite Grenzfläche (36, 38) in unterschiedliche Richtungen geneigt sind, so dass aus dem Strahlengang (14) herausreflektierte Strahlenbündel (40, 42) den Strahlengang (14) in unterschiedliche Richtungen verlassen.

6. Optisches System (2) nach einem der Ansprüche 1 bis 5, **dadurch gekennzeichnet, dass** das zumindest eine Prisma (30, 32, 34) der Prismengruppe (24) ein gerades Prisma (30, 32, 34) ist, welches zwei optisch wirksame Prismenflächen (54, 54`), die einen spitzen Winkel α einschließen, und eine dem spitzen Winkel α gegenüberliegende, optisch nicht wirksame Fläche (56) umfasst, wobei eine erste Ebene (E1), in der eine erste optisch wirksame Prismenfläche (54) liegt, eine zweite Ebene (E2), in der eine zweite optisch wirksame Prismenfläche (54') liegt, und eine dritte Ebene (E3), in der die Fläche (56) liegt, gemeinsam ein Dreieck einschließen, welches zumindest bereichsweise eine Grundfläche des Prismas ist.

7. Optisches System (2) nach Anspruch 6, **dadurch gekennzeichnet, dass** die Prismengruppe (24) zumindest ein erstes Prisma (30) und ein zweites Prisma (32) umfasst, wobei das erste und das zweite Prisma (30, 32) so angeordnet sind, dass ein erster spitzer Winkel α des ersten Prismas (30) und ein zweiter spitzer Winkel β des zweiten Prismas (32) auf einander gegenüberliegenden Seiten der Prismengruppe (24) liegen.

8. Optisches System (2) nach Anspruch 7, **dadurch gekennzeichnet, dass** das erste und das zweite Prisma (30, 32) in Lichteinfallsrichtung hintereinander angeordnet sind, wobei das erste Prisma (30) ein rechtwinkliges Prisma ist.

9. Optisches System (2) nach einem der Ansprüche 1 bis 8, **dadurch gekennzeichnet, dass** die Prismengruppe (24) ein Teil der distalen optischen Baugruppe (10) ist, wobei insbesondere die distale optische Baugruppe (10) eine Eintrittslinse (26) umfasst und die Prismengruppe (24) in Lichteinfallsrichtung unmittelbar auf die Eintrittslinse (26) folgend angeordnet ist.

10. Chirurgisches Instrument, insbesondere Endoskop, mit einem optischen System (2) nach einem der Ansprüche 1 bis 9.

## Claims

1. An optical system (2) comprising a distal optical assembly (10), a proximal optical assembly (12) and an image sensor (18), wherein the distal and proximal optical assemblies (10, 12) define a beam path (14), and the distal optical assembly (10) couples incident beams of light (6, 6`) from a field of view (20) located in an object space (4) in the proximal optical assembly (12), and the proximal optical assembly (12) directs these beams of light (6, 6`) onto a light-sensitive surface (19) of the image sensor(18), **characterized by** a group of prisms (24) arranged in the beam path (14), with at least one prism (30, 32, 34), and wherein the prism group (24) limits the field of view (20) of the optical system (2) on at least one side, wherein the at least one prism (30, 32, 34) includes a boundary surface (36, 38) at which incident beams of light (6", 6‴) from outside the field of view (20) are reflected out of the beam path (14) with total reflection, wherein the prism group (24) comprises at least a first and a second prism (30, 32), wherein the first prism (30) comprises the boundary surface (36) at which total reflection occurs and the second prism (32) is arranged abutting this boundary surface (36), wherein a first air gap (46) is present between the first prism (30) and the second prism (32).

2. The optical system (2) according to claim 1, **characterized in that** the beams of light (40, 42) reflected out enter the distal optical assembly (10) on one side of the field of view (20), at which the prism group (24) of the field of view (20) is limited.

3. The optical system (2) according to claim 1 or 2, **characterized in that** the prism group (24) also includes a third prism (34), wherein the first to third prism (30, 32, 34) are designed having mirror symmetry with respect to a vertical plane (52) perpendicular to an optical axis (16) of the prism group (24).

4. The optical system (2) according to claim 3, **characterized in that**, in the direction of incident light, the prism group (24) comprises a first rectangular prism as a first prism (30), and isosceles prism as a second prism (32) and a second rectangular prism as a third prism (34) one after another, wherein the second prism (32) comprises a second boundary surface (38) at which total reflection takes place, and the third prism (34) is arranged abutting this second boundary surface (38), wherein a second air gap (50) is present between the second prism (32) and the third prism (34).

5. The optical system (2) according to claim 4, **characterized in that** the first and second boundary surface (36, 38) are inclined in different directions, so that the beams of light (40, 42) reflected out of the beam path (14) leave the beam path (14) in different directions.

6. The optical system (2) according to one of the claims 1 to 5, **characterized in that** the at least one prism (30, 32, 34) of the prism group (24) is a straight prism (30, 32, 34) which comprises two optically effective prism surfaces (54, 54') which enclose an acute angle α and an optically non-effective surface (56) opposite the acute angle α, wherein a first plane (E1) in which a first optically effective prism surface (54) lies, a second plane (E2) in which a second optically effective prism surface (54`) lies, and a third plane (E3) in which the surface (56) lies together enclose a triangle which is a base surface of the prism at least in an area.

7. The optical system (2) according to claim 6, **characterized in that** the prism group (24) comprises at least a first prism (30) and a second prism (32), wherein the first and second prism (30, 32) are arranged such that a first acute angle α of the first prism (30) and a second acute angle β of the second prism (32) lie on mutually opposing sides of the prism group (24).

8. The optical system (2) according to claim 7, **characterized in that** the first and second prism (30, 32) are arranged one after another in the direction of incident light, wherein the first prism (30) is a rectangular prism.

9. The optical system (2) according to one of the claims 1 to 8, **characterized in that** the prism group (24) is a part of the distal optical assembly (10), wherein the distal optical assembly (10) particularly comprises an entry lens (26) and the prism group (24) is arranged directly after the entry lens (26) in the direction of incident light.

10. A surgical instrument, particularly an endoscope, with an optical system (2) according to one of the claims 1 to 9.

## Revendications

1. Système optique (2), comprenant un ensemble optique distal (10), un ensemble optique proximal (12) et un capteur d'image (18), les ensembles optiques distal et proximal (10, 12) définissant un trajet de faisceaux (14) et l'ensemble optique distal (10) couplant dans l'ensemble optique proximal (12) des faisceaux lumineux (6, 6') incidents depuis un champ de vision (20) situé dans l'espace objet (4) et l'ensemble optique proximal (12) dirigeant ces faisceaux lumineux (6, 6') sur une surface photosensible (19) du capteur d'image (18), **caractérisé par** un groupe de prismes (24) disposé dans le trajet des faisceaux (14) et comportant au moins un prisme (30, 32, 34), le groupe de prismes (24) délimitant le champ de vision (20) du système optique (2) sur au moins un côté, ledit au moins un prisme (30, 32, 34) comprenant une surface limite (36, 38) sur laquelle des rayons lumineux (6", 6‴) incidents depuis l'extérieur du champ de vision (20) sont réfléchis hors du trajet des faisceaux (14) avec une réflexion totale, le groupe de prismes (24) comprenant au moins un premier et un deuxième prismes (30, 32), le premier prisme (30) comprenant la surface limite (36) sur laquelle a lieu la réflexion totale et le deuxième prisme (32) étant disposé de manière adjacente à cette surface limite (36), un premier espace d'air (46) étant présent entre le premier prisme (30) et le deuxième prisme (32).

2. Système optique (2) selon la revendication 1, **caractérisé en ce que** les rayons lumineux réfléchis vers l'extérieur (40, 42) sont incidents dans l'ensemble optique distal (10) sur un côté du champ de vision (20) sur lequel le groupe de prismes (24) délimite le champ de vision (20).

3. Système optique (2) selon la revendication 1 ou la revendication 2, **caractérisé en ce que** le groupe de prismes (24) comprend en outre un troisième prisme (34), les premier à troisième prismes (30, 32, 34) étant construits de manière symétrique par rapport à un plan médian (52) situé perpendiculairement à un axe optique (16) du groupe de prismes (24).

4. Système optique (2) selon la revendication 3, **caractérisé en ce que** le groupe de prismes (24) comprend, dans la direction d'incidence de la lumière, successivement un premier prisme à angle droit en tant que premier prisme (30), un prisme isocèle en tant que deuxième prisme (32) et un deuxième prisme à angle droit en tant que troisième prisme (34), le deuxième prisme (32) comprenant une deuxième surface limite (38) sur laquelle une réflexion totale a lieu, et le troisième prisme (34) étant disposé de manière adjacente à cette deuxième surface limite (38), un deuxième espace d'air (50) étant présent entre le deuxième prisme (32) et le troisième prisme (34).

5. Système optique (2) selon la revendication 4, **caractérisé en ce que** la première et la deuxième surface limite (36, 38) sont inclinées dans des directions différentes, de sorte que les faisceaux de rayons (40, 42) réfléchis à partir du trajet des faisceaux (14) quittent le trajet des faisceaux (14) dans des directions différentes.

6. Système optique (2) selon l'une des revendications 1 à 5, **caractérisé en ce que** ledit au moins un prisme (30, 32, 34) du groupe de prismes (24) est un prisme droit (30, 32, 34) qui comprend deux surfaces de prisme (54, 54') optiquement actives qui forment un angle aigu α et une surface optiquement non active (56) opposée à l'angle aigu a, un premier plan (E1), dans lequel se trouve une première surface de prisme (54) optiquement active, un deuxième plan (E2), dans lequel se trouve une deuxième surface de prisme (54') optiquement active, et un troisième plan (E3), dans lequel se trouve la surface (56), délimitent ensemble un triangle qui est au moins par zones une surface de base du prisme.

7. Système optique (2) selon la revendication 6, **caractérisé en ce que** le groupe de prismes (24) comprend au moins un premier prisme (30) et un deuxième prisme (32), le premier et le deuxième prismes (30, 32) étant disposés de telle sorte qu'un premier angle aigu α du premier prisme (30) et un deuxième angle aigu β du deuxième prisme (32) se trouvent sur des côtés opposés du groupe de prismes (24).

8. Système optique (2) selon la revendication 7, **caractérisé en ce que** les premier et deuxième prismes (30, 32) sont disposés l'un derrière l'autre dans la direction d'incidence de la lumière, le premier prisme (30) étant un prisme à angle droit.

9. Système optique (2) selon l'une des revendications 1 à 8, **caractérisé en ce que** le groupe de prismes (24) fait partie de l'ensemble optique distal (10), l'ensemble optique distal (10) comprenant notamment une lentille d'entrée (26) et le groupe de prismes (24) étant disposé immédiatement après la lentille d'entrée (26) dans la direction d'incidence de la lumière.

10. Instrument chirurgical, notamment endoscope, comprenant un système optique (2) selon l'une des revendications 1 à 9.
